# EUROPEAN PATENT SPECIFICATION

(11) **EP 0 559 625 B1**
(45) Date of publication and mention of the grant of the patent: **27.08.1997**
(21) Application number: 93830083.7
(22) Date of filing: 01.03.1993
(51) Int. Cl.: C07C 229/22, A61K 31/22

(54) **Esters of L-carnitine and acyl L-carnitine endowed with muscle relaxant activityselective on gastrointestinal tract and pharmaceutical compositions containing same**
Ester von L-Carnitin und Acyl-L-Carnitin, mit entspannender Wirkung, selektiv auf den Gastrointestinaltrakt und sie enthaltende pharmazeutische Zusammensetzungen
Esters de L-carnitine et acyl-L-carnitine dotés d'une activité relaxante des muscles, sélective dans le tractus gastro-intestinel et compositions pharmaceutiques les contenant

(30) Priority: 02.03.1992 IT RM920138
(43) Date of publication of application: 08.09.1993
(73) Proprietor: Sigma-Tau Industrie Farmaceutiche Riunite S.p.A., 00144 Roma (IT)
(72) Inventor: Scafetta, Nazareno, I-00040 Pavona di Albano RM (IT); Santaniello, Mosé, I-80026 Casoria NA (IT); Tinti, Maria Ornella, I-00182 Roma RM (IT); Misiti, Domenico, I-00199 Roma (IT); Stasi, Maria Antonietta, I-00040 Torvaianica RM (IT); Vesci, Loredana, I-00154 Roma RM (IT); Corsico, Nerina, I-20134 Milano Mi (IT)
(74) Representative: Cavattoni, Fabio

(56) References cited:
- EP-A- 0 167 115
- EP-A- 0 552 138
- GB-A- 2 051 779
- GB-A- 2 076 816
- BIOMEDICAL AND ENVIRONMENTAL MASS SPECTROMETRY vol. 18, no. 9, September 1989, CHICHESTER, GB pages 668 - 72 K.N. CHENG ET. AL. 'Characterisation of Acylcarnitines as their Isobutyl Ester Derivatives using Fast Atom Bombardment Mass Spectrometry and Constant Neutral Loss Scan.'
- CHEMICAL ABSTRACTS, vol. 67, no. 23, 4 December 1967, Columbus, Ohio, US; abstract no. 106315e, C. MEZEI ET. AL. 'The formation of lipid bound carnitine derivatives in the fat body of Phormia Regina' page 10008;column 2; & 8TH COLLECTIVE INDEX & J. INSECT PHYSIOL. vol. 13, no. 10, pages 1489-99

## Description

The present invention relates to esters of L-carnitine and acyl L-carnitine of formula (I): 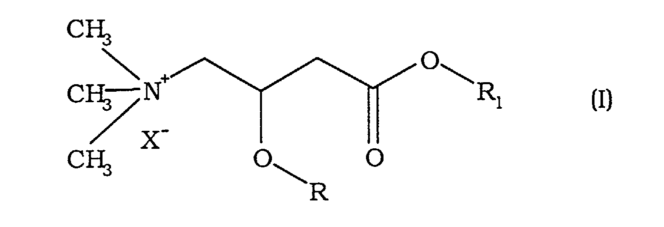 wherein:
R is hydrogen or is a straight or branched, saturated or unsaturated acyl group having 2 to 26 carbon atoms;
R₁ is a straight or branched, saturated or unsaturated alkyl group having 4 to 26 carbon atoms; and
X⁻ is the anion of a pharmacologically acceptable acid, provided that
   (1) if R is hydrogen, R₁ is not isobutyl;
   (2) if R is C₂-C₁₀ acyl or linoleyl, R₁ is not butyl or isobutyl; and
   (3) if R is C₂-C₁₆ acyl, R₁ is not C₁₁-C₁₆ alkyl.

These compounds are endowed with calcium-antagonist activity. However, unlike known calcium antagonists (such as e.g. Diltiazem, Verapamil and Nitrendipina) it was surprisingly found that the compounds of formula (I) exhibit potent muscle relaxant activity affecting the gastrointestinal tract selectively, with no effect whatsoever on the cardiovascular tract. Moreover, they exhibit remarkable muscle relaxant activity on the intestinal contractility brought about by contracture-inducing drugs having different mode of action (e.g. acetylcholine).

Esters of L-carnitine and acyl L-carnitine are already known.

For instance, GB 2 076 816 discloses carnitine isobutyl ester and carnitine isopropyl ester as intermediates in the preparation of ethoxyacetyl carnitine isobutyl ester and 3-ethoxypropionyl carnitine isopropyl ester, respectively, which are therapeutically useful for the treatment of cardiac arrhythmias and hyperlipidaemias.

EP 0 167 115 discloses the condensation products of L-carnitine methyl and ethyl ester with potassium aspartate, succinate or glutarate which are useful for the treatment of myocardial anoxia, ischemia and cardiac failure.

Esters of acyl L-carnitines of formula 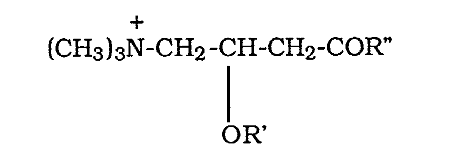 wherein R' is acyl and R" is an alkoxy radical are disclosed in:
- GB 2 051 779, wherein R' is acetyl, propionyl or butyryl, optionally halogen-substituted and R" is an alkoxy having 1-4 carbon atoms; the esters are useful for the treatment of myocardial hypocontractility and as antidepressants;
- EP 0 552 137 and EP 0 552 138, wherein R'is a straight or branched acyl group having 2 to 16 carbon atoms and R" is a straight alkoxy having 8-16 carbon atoms; the esters are endowed with antibacterial and antimycotic activity.

The compounds of formula (I) are useful as active ingredients in orally or parenterally administrable pharmaceutical compositions, for treating adaptive colitis syndromes as well as all those pathologies wherein an increase of intestinal contractility and/or motility can be found.

In compounds of formula (I), when R is a straight saturated acyl group having 2 to 26 carbon atoms, it is preferably selected from acetyl, propionyl, butyryl, palmitoyl, undecanoyl and hexanoyl;
when R is branched acyl, it is preferably selected from isobutyryl, isovaleryl, isocaproyl and 2-methylhexanoyl;
when R is unsaturated acyl, it is preferably 10-undecenoyl.

As regards R₁ (alkyl group having 4 to 26 carbon atoms), when R₁ is straight saturated alkyl, it is preferably selected from n-butyl, n-heptyl, n-undecyl and n-hexacosyl;
when R₁ is branched alkyl, it is preferably selected from isobutyl, isooctyl, hexylmethylcarbyl, ethylpentylcarbyl, ethylhexylcarbyl, decylmethylcarbyl, dipentylcarbyl and methylnonylcarbyl;
when R₁ is unsaturated alkyl, it is preferably pentylvinylcarbyl or 10-undecenyl.

The anion X⁻ of the pharmacologically acceptable acid is preferably selected from chloride; bromide, iodide; aspartate, particularly acid aspartate; citrate, particularly acid citrate; tartrate; phosphate, particularly acid phosphate; fumarate, particularly acid fumarate; glycerophosphate; glucosephosphate; lactate; maleate, particularly acid maleate; orotate; oxalate, particularly acid oxalate; sulphate, particularly acid sulphate; trichloroacetate; trifluoroacetate and methansulphonate.

Compounds of formula (I) which are particularly preferred are Acetyl L-carnitine heptyl ester chloride, Isobutyryl L-carnitine heptyl ester chloride, Isobutyryl L-carnitine n-butyl ester chloride, Isovaleryl L-carnitine n-butyl ester chloride, Isovaleryl L-carnitine isobutyl ester chloride, Isovaleryl L-carnitine heptyl ester chloride, L-carnitine heptyl ester chloride, Acetyl L-carnitine hexacosyl ester chloride, Isovaleryl L-carnitine undecyl ester chloride, and Isobutyryl L-carnitine undecyl ester chloride.

It has, furthermore, been found that also the foregoing known esters of L-carnitine and acyl L-carnitine exhibit calcium-antagonist activity. Consequently, the present invention also relates to the use of an ester of L-carnitine or acyl L-carnitine of formula (I): 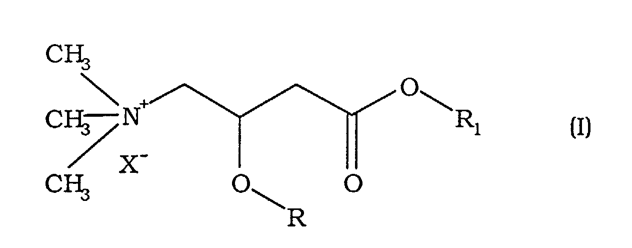 wherein
R is hydrogen or is a straight or branched, saturated or unsaturated acyl group having 2 to 26 carbon atoms;
R₁ is a straight or branched, saturated or unsaturated alkyl group having 4 to 26 carbon atoms; and
X⁻ is the anion of a pharmacologically acceptable acid,
for producing a medicament for treating adaptive colitis syndromes and pathological conditions characterized by increased intestinal contractility and/or motility.

The esters of formula (I) may be prepared following two distinct synthesis processes. The first process (illustrated in the Synthesis Scheme 1) comprises the steps consisting of:
(a) halogenating an acyl L-carnitine with a halogenating agent such as thionyl chloride and oxalyl chloride (molar ratio comprised between 1:1 and 1:4) in an anhydrous organic inert solvent such as acetonitrile or methylene chloride at a temperature comprised between 0°C and 30°C for 1-4 hours, concentrating the raw reaction product and using it in the following step;
(b) dissolving the acid chloride of step (a) in an anhydrous organic inert solvent such as acetonitrile or methylene chloride and adding the alcohol diluted in the same solvent at a ratio comprised between 1:1 and 1:2 at temperatures comprised between 0°C and 30°C for 2-10 hours, concentrating the solution and, if needed, purifying the compound by chromatography on silica gel; and
(c) eluting the product dissolved in water or in an organic solvent on a strongly basic ion exchange resin such as Amberlite IRA 402 or on a weakly basic ion exchange resin such as Amberlist A 21, activated with the desired HX acid and isolating the final product by lyophilization or concentration.

The second process (illustrated in the Synthesis Scheme 2) comprises the steps consisting of:
(a') reacting carnitine or an acyl carnitine inner salt with the relevant alkyl halogenide (preferably bromide or iodide) in an organic anhydrous inert solvent at a temperature comprised between 30°C and 60°C for 8-24 hours and then isolating the resulting compound by concentration;
(b') acylating the ester obtained in step (a') with the desired acid chloride by known techniques, in case the starting compound in step (a') is carnitine;
(c') eluting an aqueous or alcoholic solution of the compound of step (a') or (b') on an ion exchange resin such as Amberlite IRA 402 or Amberlist A 21 activated with the desired HX acid.

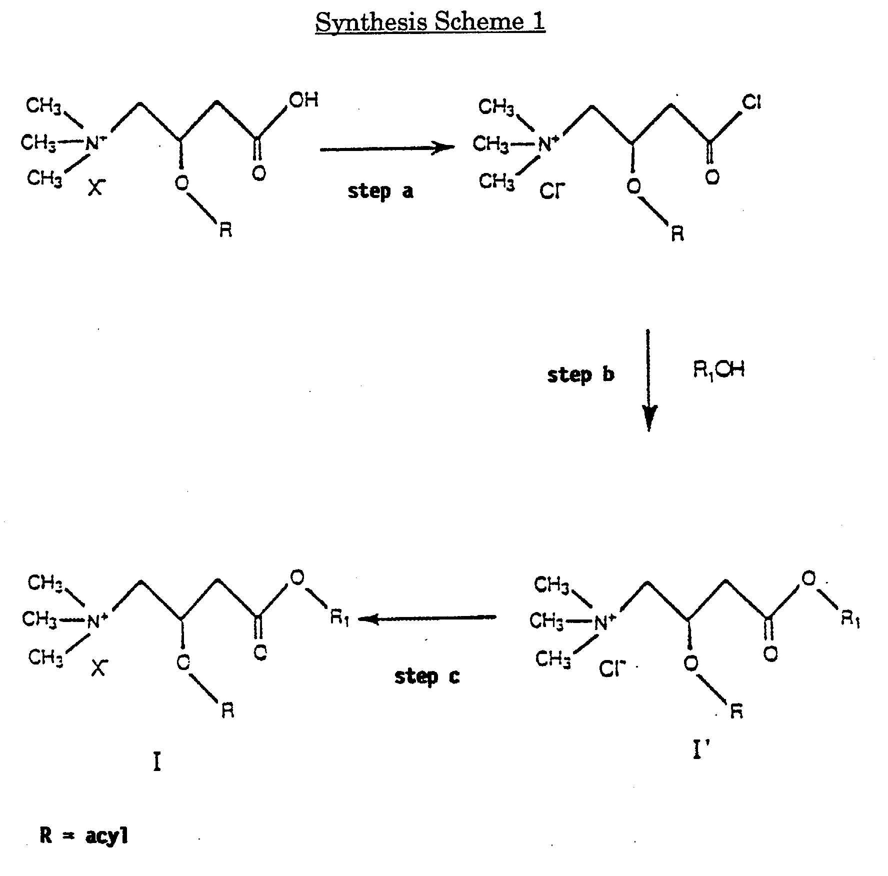

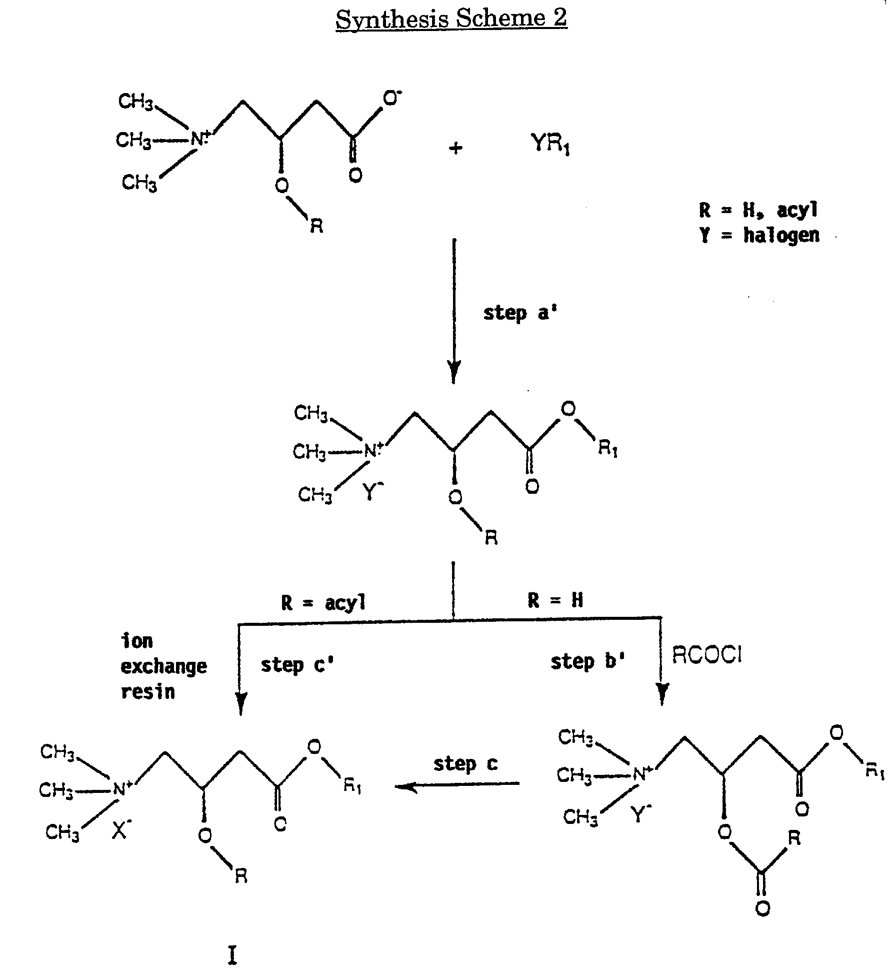

### Example 1

### Preparation of acetyl L-carnitine heptyl ester chloride (ST 904)

### Step (a'): Preparation of acetyl L-carnitine heptyl ester iodide.

Acetyl L-carnitine inner salt (30 g; 0.148 moles) was suspended in 50 ml anhydrous CH₃CN. To this mixture heptyl iodide (24.5 ml; 0.149 moles) was added. The resulting solution was reacted for 12 hours at 50°C and then concentrated under vacuum. An oily residue was obtained which was used as such in the next step.

### Step (c'):

The raw products of step a' was dissolved in H₂O and eluted through a column of 600 ml Amberlite IRA 402 resin activated in Cl- form. The collected eluate was lyophilized and 45 g of a vitreous solid product were obtained. Yield 91%.
[α]²⁵_{D} =-11.2 (c=0.5% CHCl₃)
E.A. C₁₆H₃₂ClNO₄

| | C% | H% | N% | Cl% |
|---|---|---|---|---|
| calculated (anhydrous) | 56.96 | 9.49 | 4.15 | 10.52 |
| found | 54.63 | 10.17 | 4.06 | 9.74 |

H₂O 3.8%
HPLC
Column: Spherisorb Cl 5 µm
t: 50°C
Flow rate: 1 ml/min
Rt: 17.44 min
NMR D₂O δ 5.6 (1H,m, 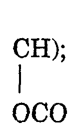 4.1(2H,t,OCH₂); 4.0-3.7 (2H,m,N⁺CH₂₋); 3.3 (9H,s,(CH₃)₃N⁺); 2.8 (2H,dd,CH₂COO); 2.1 (3H,s,COCH₃); 1.3 (10H,m, (CH₂)₅); 0.9 (3H,m,CH₂CH₃)

### Example 2

### Preparation of isobutyryl L-carnitine heptyl ester chloride (ST 713)

### Step (a): Preparation of the acid chloride of isobutyryl L-carnitine chloride.

Isobutyryl L-carnitine chloride was suspended in oxalyl chloride (16 ml; 0.095 moles). The mixture was kept under stirring at room temperature for 6 hours. Anhydrous ethyl ether was then added till complete precipitation of an oily product. The solution was concentrated and the residue washed three times with anhydrous acetone and dried under vacuum. 9 g of product were obtained. The raw product was used as such in the next step.

### Step (b): Preparation of isobutyryl L-carnitine heptyl ester chloride (ST 713).

To the acid chloride of isobutyryl L-carnitine (9 g) (prepared as shown in step a), heptanol (50 ml) was added under stirring at 0°C.

The resulting solution was kept at room temperature for 8 hours, then concentrated under vacuum to a small volume, diluted with CHCl₃ and chromatographed on silica buffered with 2% Na₂HPO₄. The column was eluted with CHCl₃ to remove the unreacted heptanol and then against CHCl₃-MeOH gradient till 100% MeOH. The oily residue thus obtained was repeatedly washed with hexane and dried under vacuum.
10.5 g of a hygroscopic oily product were obtained. Yield 89%.
TLC CHCl₃-MeOH-H₂O-IsoprOH (60-40-15-10)
RF=0.7
[α]²⁵_{D} =-16.7 (c=1% H₂O)
E.A. C₁₈H₃₆NO₄Cl

| | C% | H% | N% | Cl% |
|---|---|---|---|---|
| calculated (anhydrous) | 59.07 | 9.91 | 3.82 | 9.68 |
| found | 56.87 | 10.30 | 3.63 | 9.12 |

H₂O 4.6%
HPLC
Column: Lichrosorb BRP2 (10 µm)
Mobile phase: (NH₄)₂HPO₄ 0.05M-CH₃CN (1:1)
pH 7 with H₃PO₄
Flow rate: 2 ml/min
RT=7.44
RT=5.07 10% carnitine heptil ester
NMR CDCl₃ δ 5.5 (1H,m, 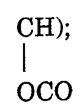 4.2-3.8 (4H,m,N⁺CH₂-; OCH₂); 3.3 (9H,s, (CH₃)₃N⁺); 2.7 (2H,m,CH₂COO); 2.4 (1H,m,COCH); 1.5-0.8 (19H,m, (CH₂)₅-CH₃;

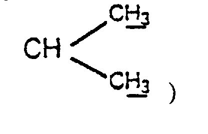

The compounds of Examples 3-7 were prepared following the procedures disclosed in Example 1.

The compounds of Examples 8-9 and 12-32 were prepared following the procedures disclosed in Example 2.

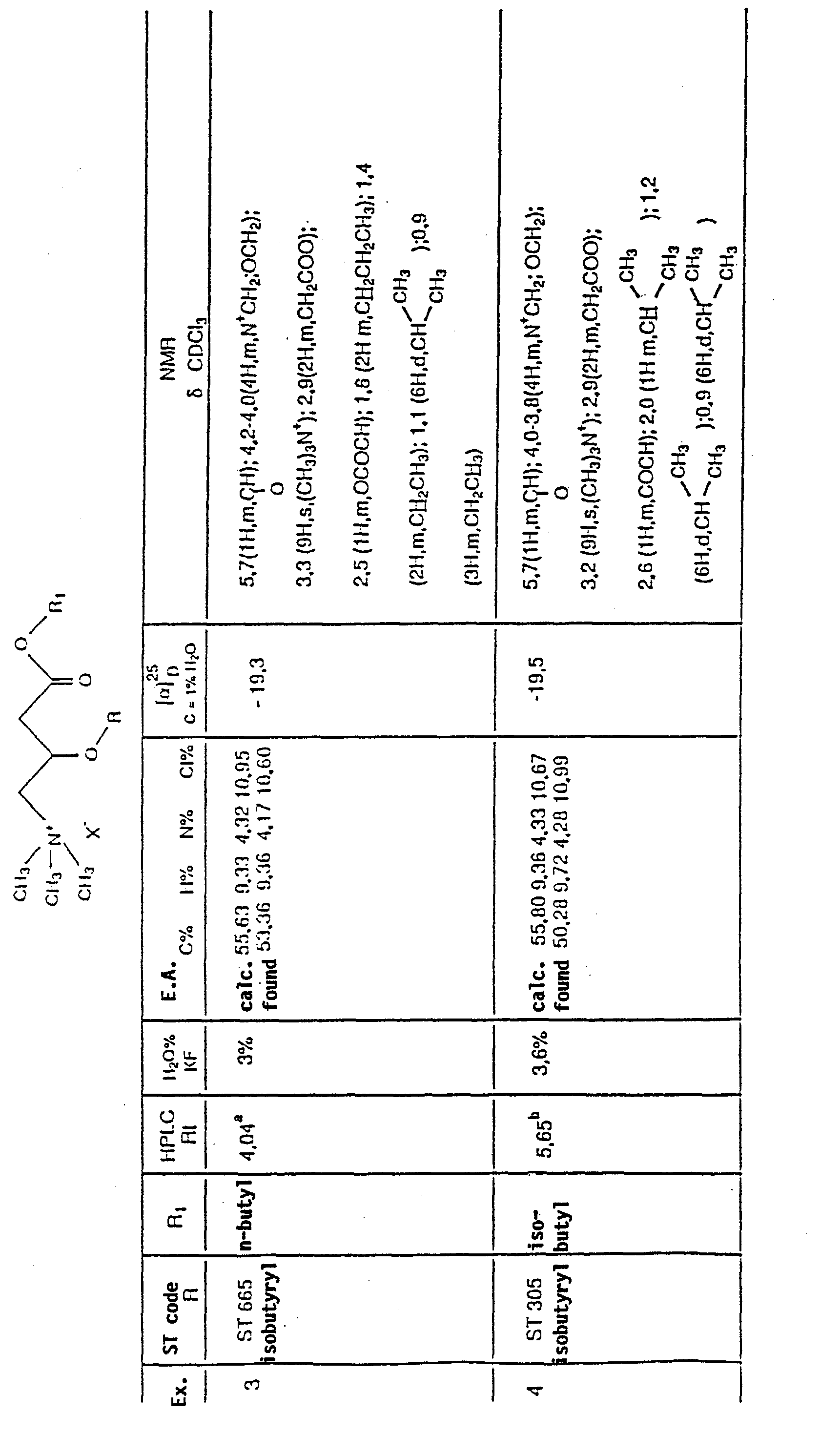

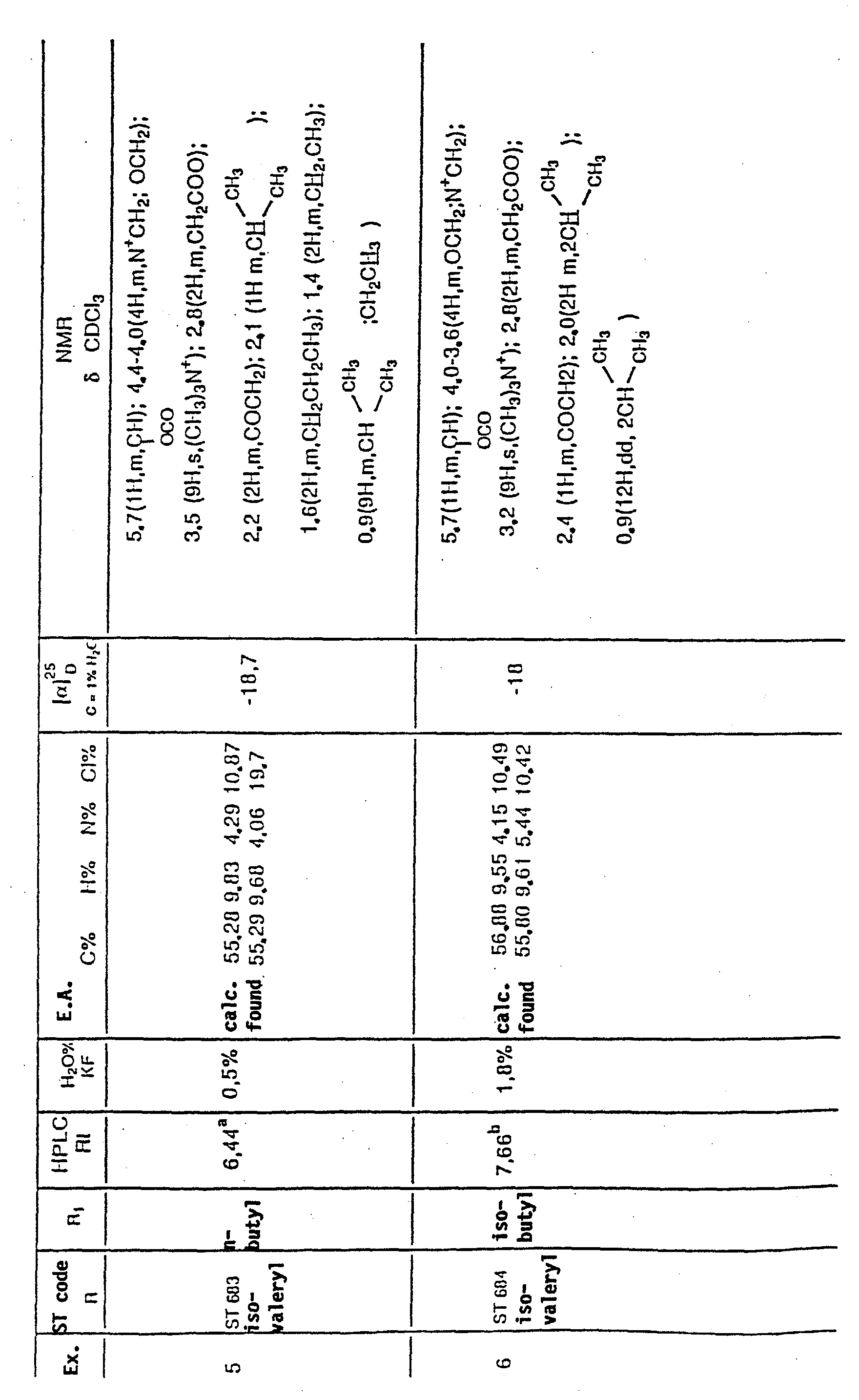

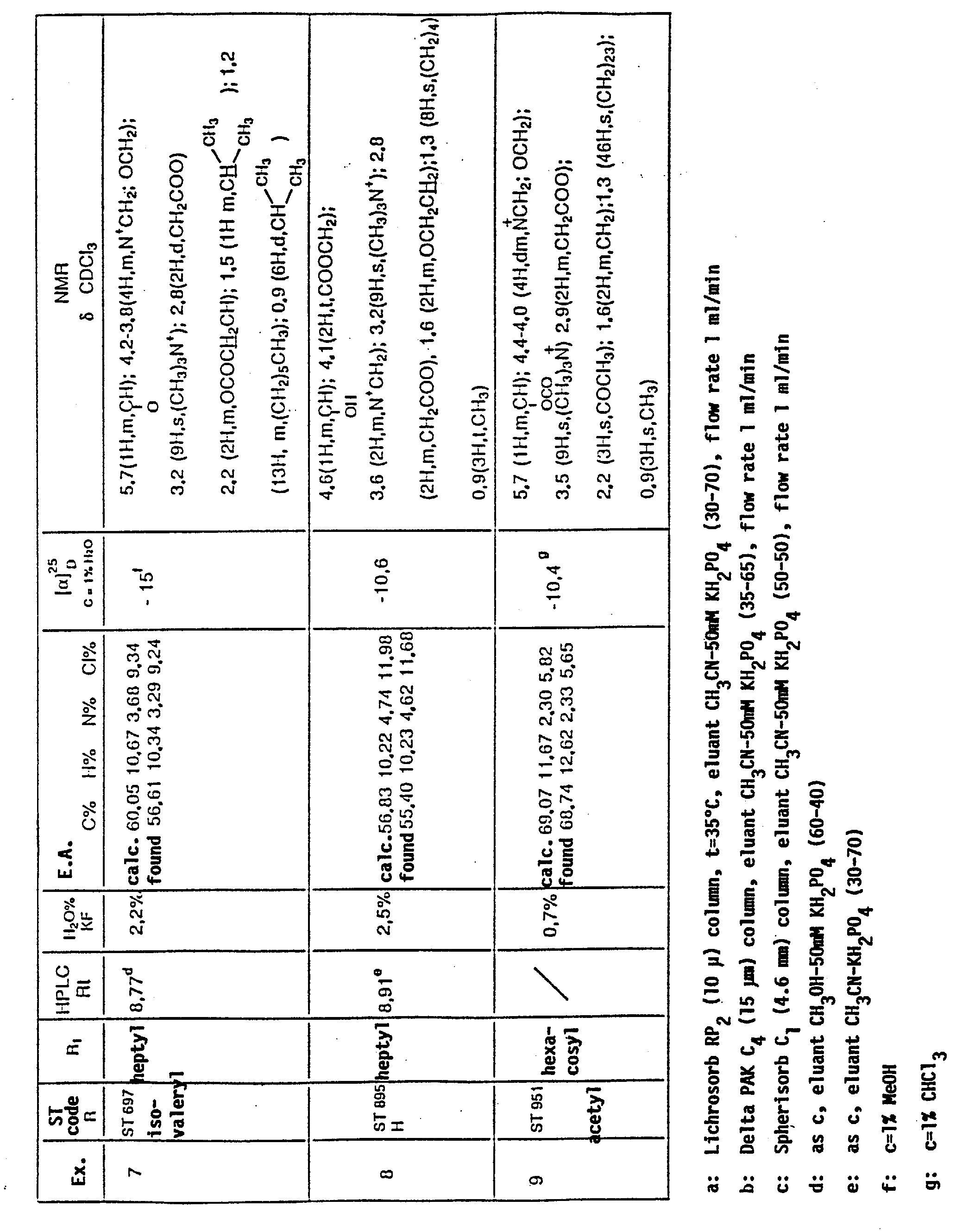

### Example 10

### Preparation ofisovaleryl L-carnitine undecylesterchloride(ST722

### Step A: Preparation of isovaleryl L-carnitine chloride acid chloride.

Isovaleryl L-carnitine chloride (30 g; 0.106 moles) was suspended in 100 ml anhydrous CH₂Cl₂.

The mixture was cooled at 0°C and oxalyl chloride (13 ml; 0.15 moles) diluted in 15 ml anhydrous CH₂Cl₂ was slowly added under stirring.

After 30 minutes at room temperature, a further amount of oxalyl chloride (19 ml; 0.21 moles) diluted in 10 ml anhydrous CH₂Cl₂ was added.

The resulting solution was kept under stirring for 2 hours at room temperature, then concentrated under vacuum.

The residue thus obtained was washed twice with anhydrous CH₂Cl₂ and concentrated under vacuum.

The raw products thus obtained was used as such in the next reaction.

### Step B: Preparation of isovaleryl L-carnitine undecyl ester chloride (ST 722).

The acid chloride previously prepared (0.106 moles) was dissolved in anhydrous CH₂Cl₂ (40 ml).

The solution was cooled at 0°C and undecylic acid (35 ml; 0.168 moles) diluted in 35 ml CH₂Cl₂ was added in a nitrogen atmosphere.

The solution was kept under stirring at room temperature for 2 hours and then concentrated under vacuum until an oily residue was obtained.

The raw reaction mixture was chromatographed on a silica gel column buffered with 2% Na₂HPO₄, eluting with CH₂Cl₂ till complete elution of undecylic alcohol and then with CH₂Cl₂-MeOH 9:1 till complete elution of the compound.

The pooled fractions were concentrated and gave 28 g of the title compound; yield 60%.
[α]²⁵_{D} =-10.5 (c=1% H₂O)
E.A. C₂₃H₄₆CINO₄

| | C% | H% | N% | Cl% |
|---|---|---|---|---|
| calculated (anhydrous) | 63.35 | 10.63 | 3.21 | 8.13 |
| found | 60.87 | 0.88 | 3.29 | 8.14 |

H₂O 2.4%
HPLC
Column: Spherisorb Cl (5 µm)
t: 50°C
Eluant: CH₃OH/50 mM KH₂PO₄ (65:35)
Flow rate: 1 ml/min
RT=14.82 min
NMR CDCl₃ δ 5.5 (1H,m, 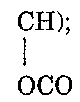 4.2-3.8 (4H,m,N⁺CH₂₋; OCH₂); 3.3 (9H,s, (CH₃)₃N⁺); 2.8(2H,m,CH₂COO); 2.2(2H,m,OCOCH₂); 1.6-1.0 (22H,m, 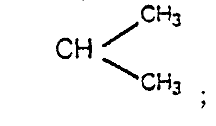 (CH₂)₉-CH₃); 0.8 (6H,d,

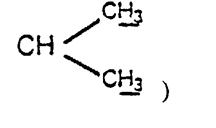

### Example 11

### Preparation ofisobutyrylL-carnitineundecylesterchloride(ST 712

The compound was prepared as described in Example 10, substituting isobutyryl L-carnitine chloride for isovaleryl L-carnitine chloride. Yield 55%.
[α]²⁵_{D} =-15.8 (c=1% H₂O)
E.A. C₂₂H₄₄O₄NCl

| | C% | H% | N% | Cl% |
|---|---|---|---|---|
| calculated (anhydrous) | 62.61 | 10.51 | 3.32 | 8.40 |
| found | 61.77 | 10.67 | 3.29 | 8.17 |

H₂O 0.8%
HPLC
Column: Spherisorb Cl (4.6 µm)
Eluant: CH₃OH/50 mM KH₂PO₄ (60:40)
Flow rate: 1 ml/min
RT=14.75 min
NMR CDCl₃ δ 5.5 (1H,m, 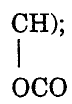 4.2-3.8 (4H,m,N⁺CH₂-; OCH₂); 3.3 (9H,s, (CH₃)₃N⁺); 2.8 (2H,m,CH₂COO); 2.5 (1H,m,COCH); 1.5-0.9 (27H,m, 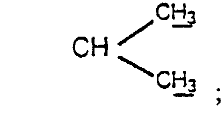 (CH₂)₉-CH₃)

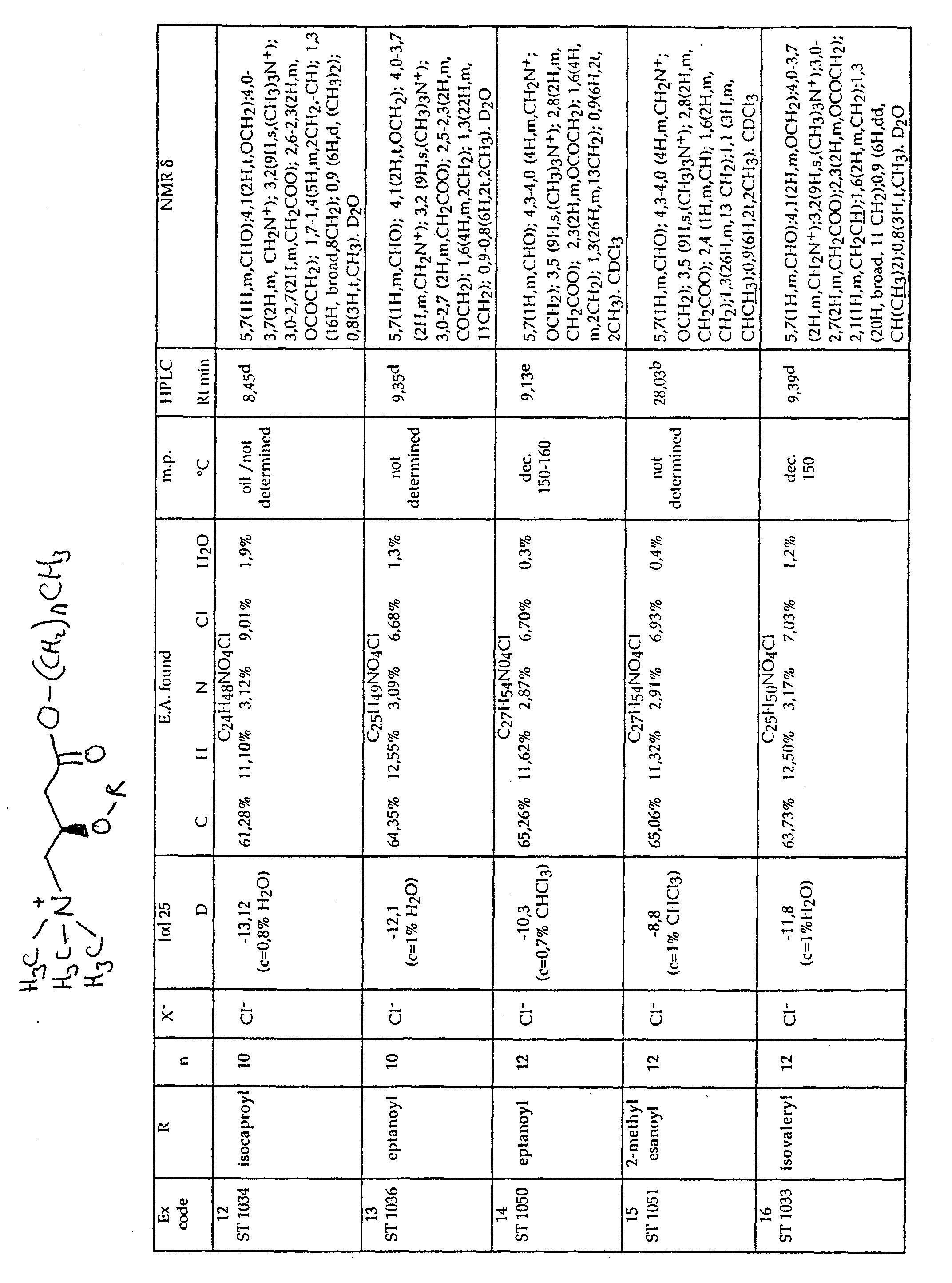

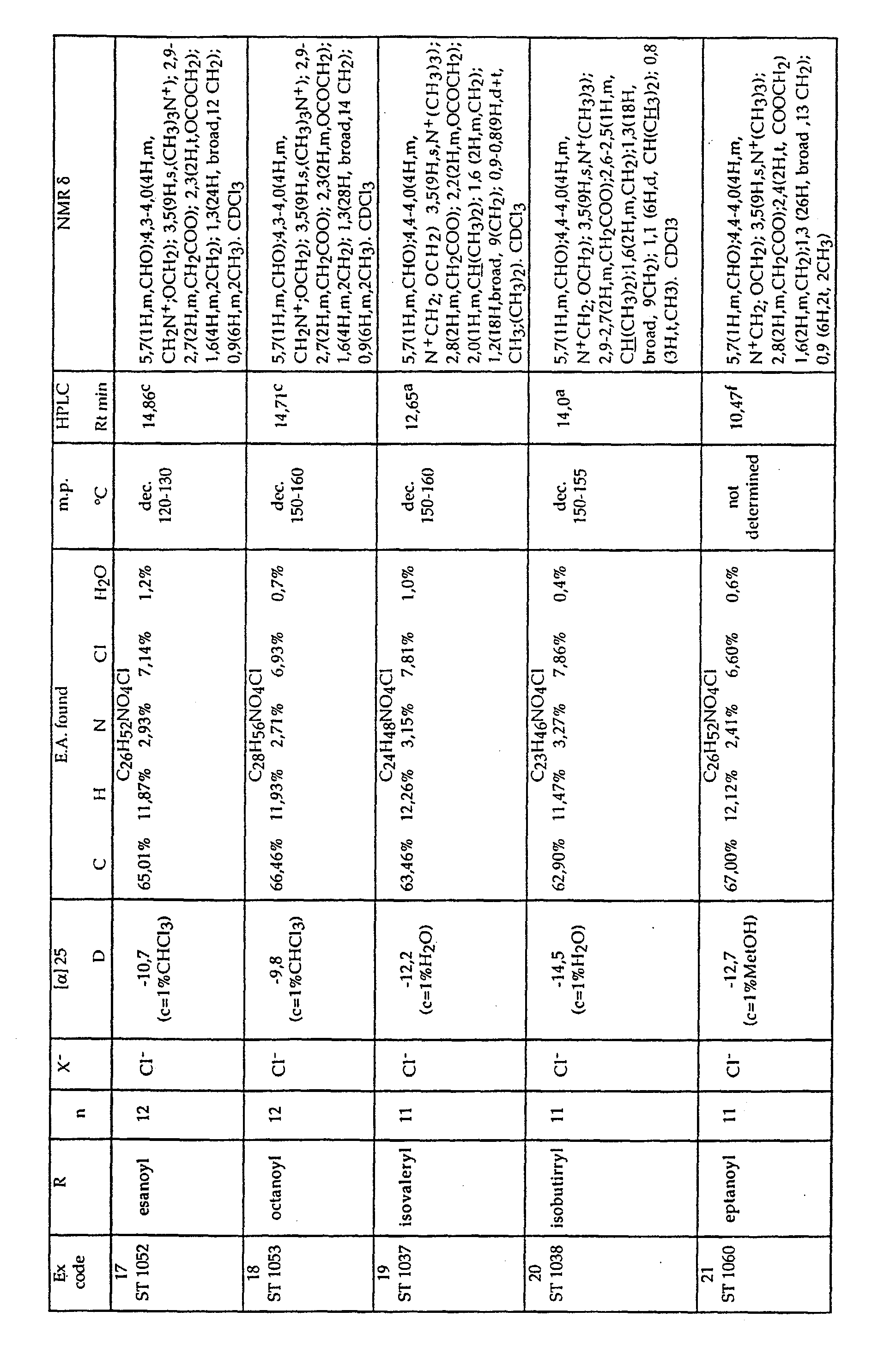

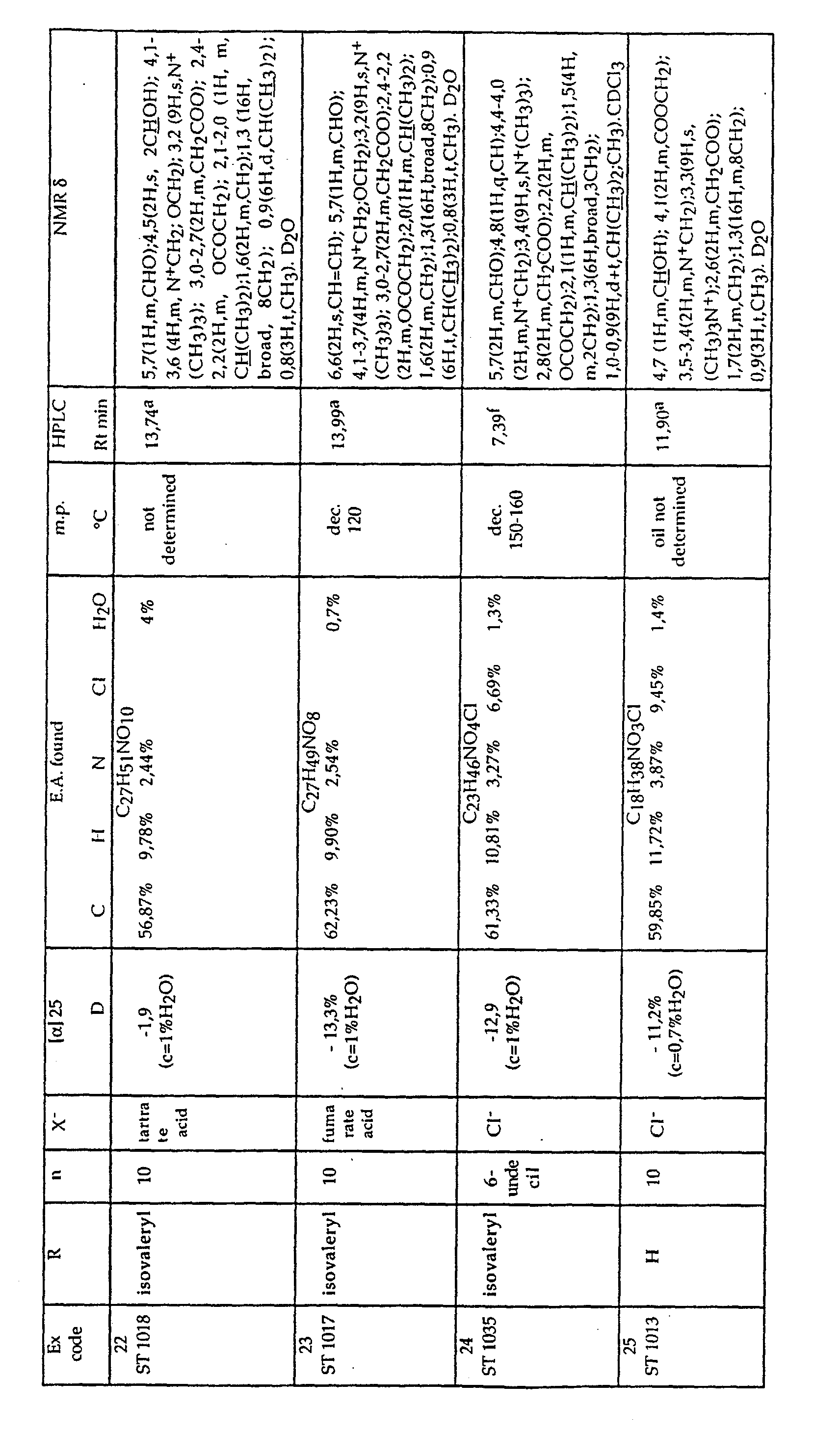

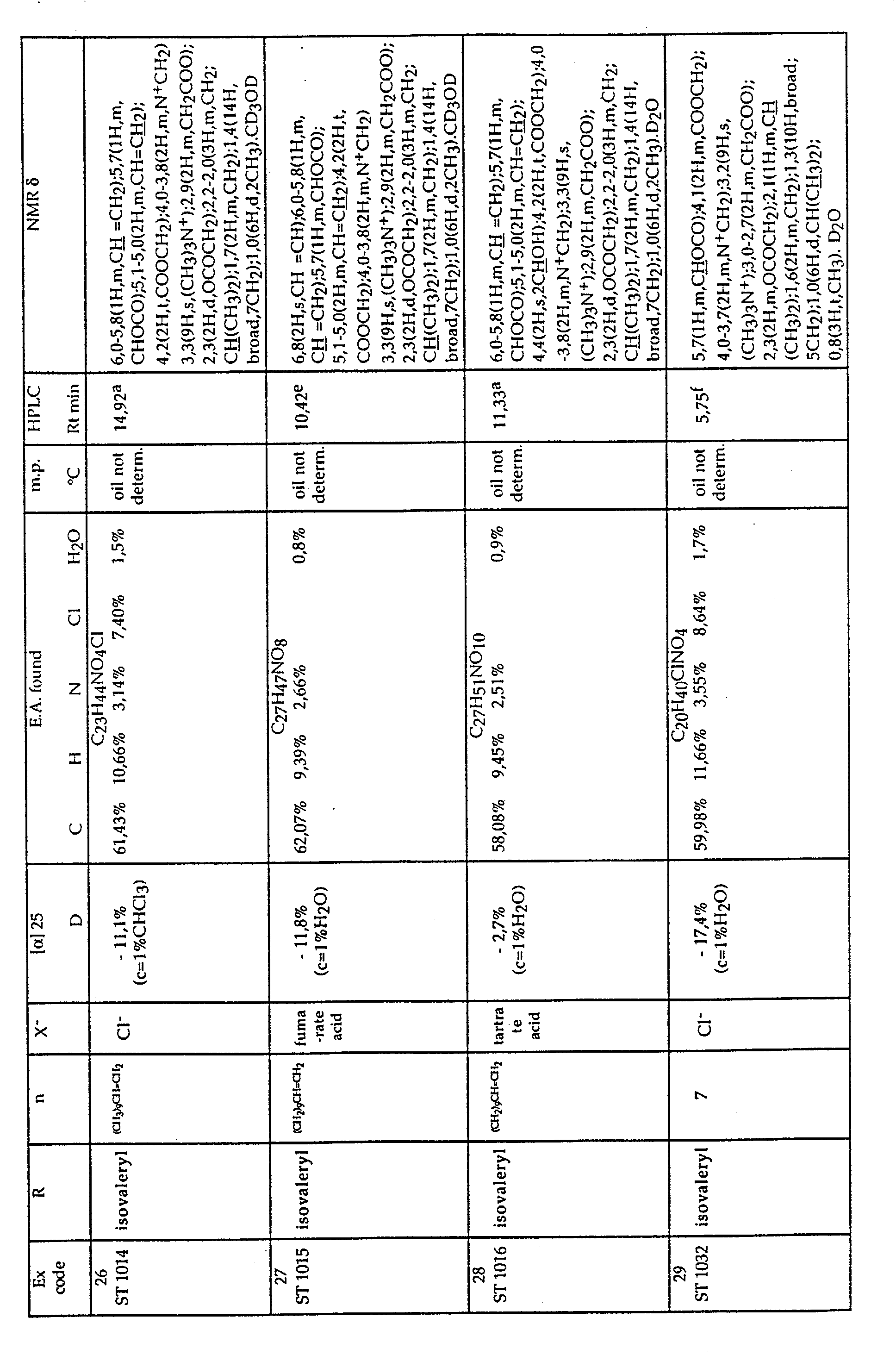

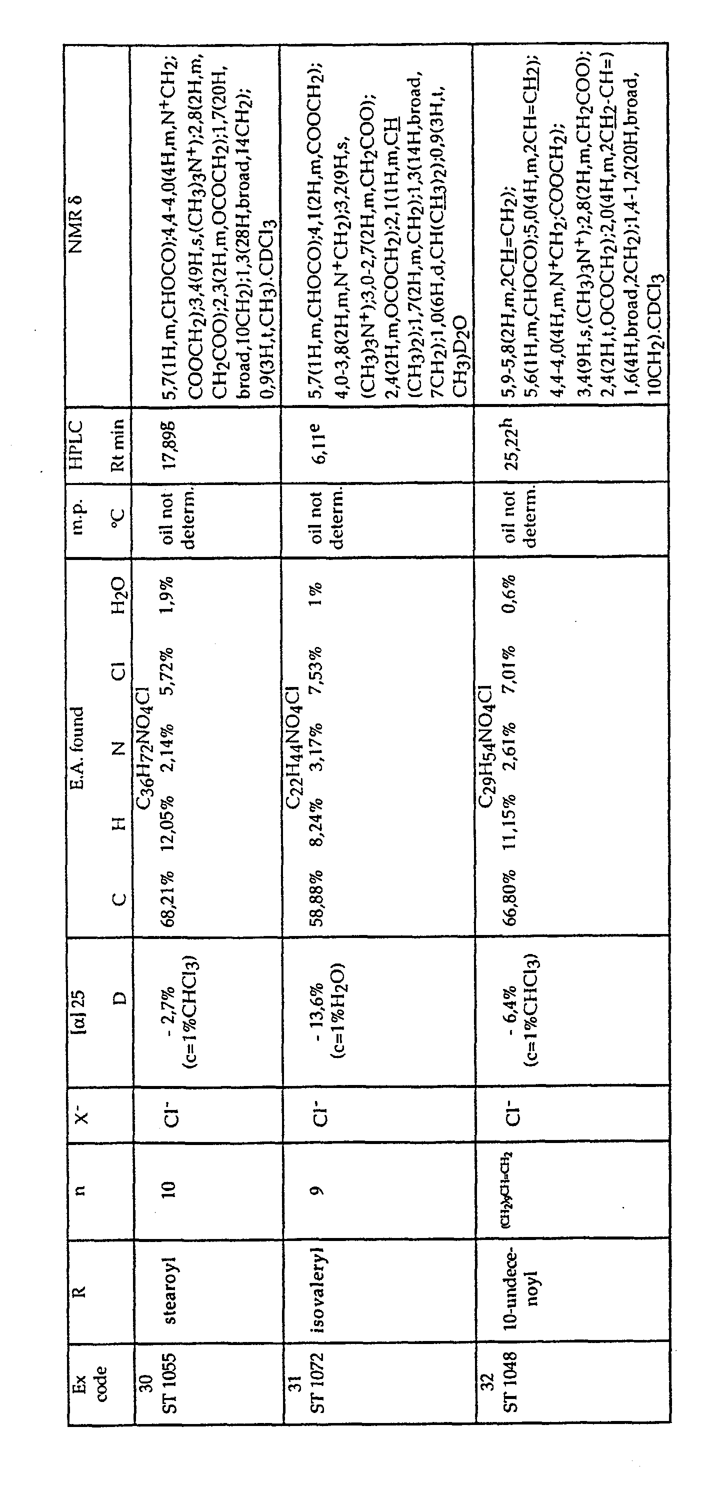

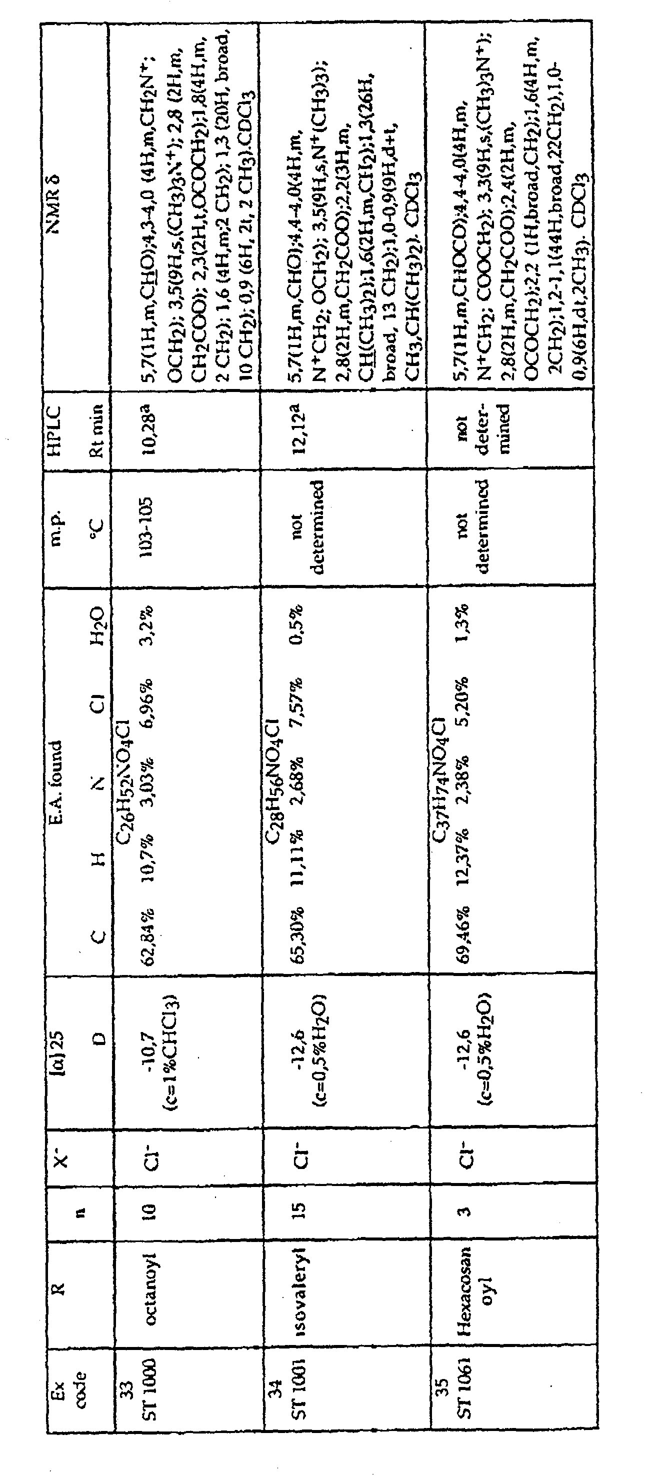

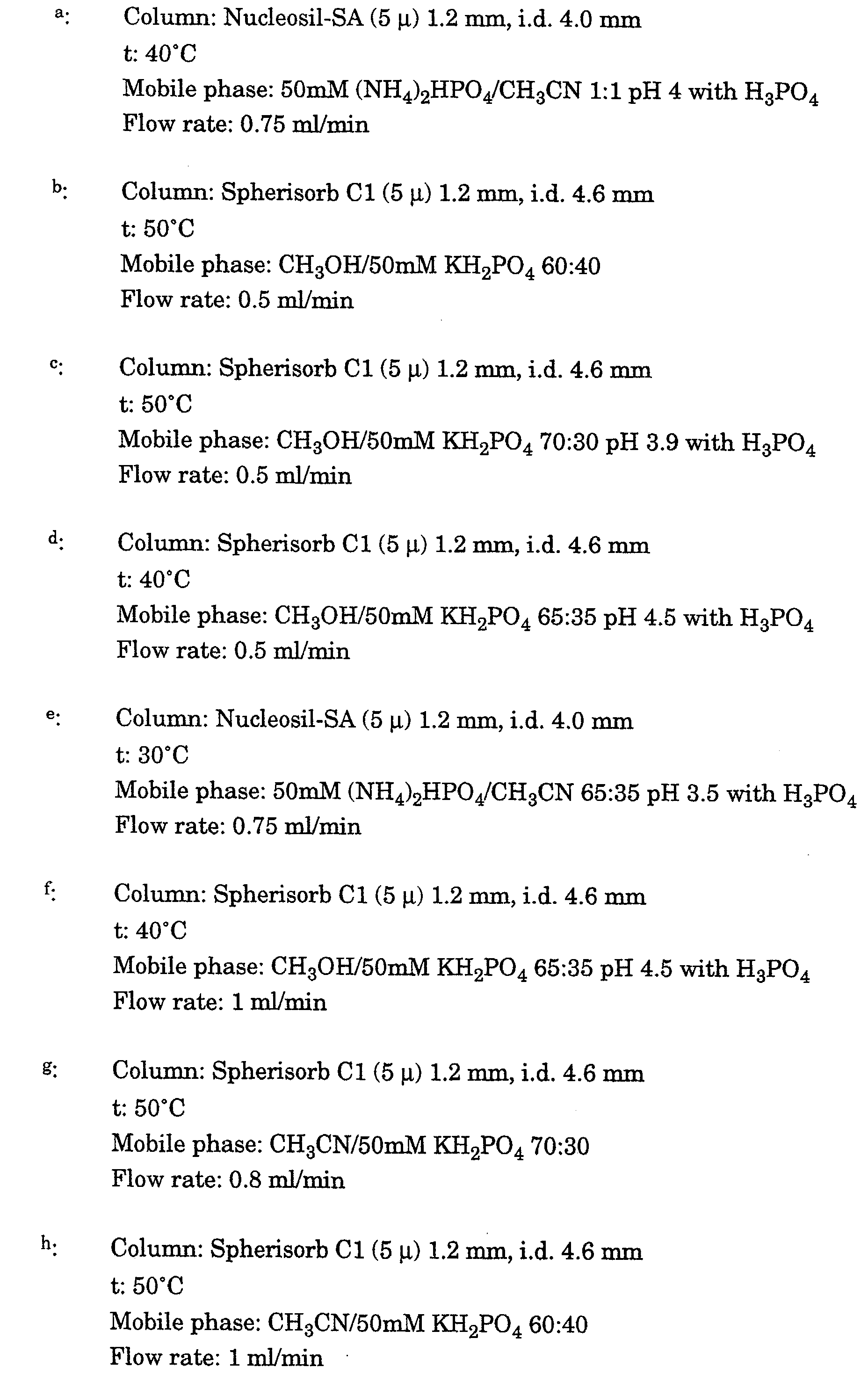

### Binding studies of ca⁺⁺ channel receptors

The "binding" of the tested compounds to the sites associated to calcium channels labeled by ³H-Nitrendipine, ³H-Verapamil and ³H-Diltiazem has been assayed in rat brain according to the method of Schoemaker H. et al. Eur. J. Pharmacol. 111, 273 (1985) and of Reynolds I.J. et al. Eur. J. Pharmacol. 95, 319 (1983).

Brain cortices are removed from decollated male CrI: CD (SD) BR rats weighing 250-300 g.

The tissues were homogenized in Tris-HCl buffer and centrifuged at 50000 x g for 10 minutes. The pellet was washed twice through a fresh buffer suspension and centrifuged at 50000 x g for 10 minutes.

After suspension of the precipitate in the incubation buffer the compounds under examination were added to the medium, starting from a concentration 10⁻⁵ M. Incubation conditions were: ³H-Nitrendipine (0.5 nM) x 60' at 30°C; ³H-Verapamil (10 µM) x 20' at 37°C; and ³H-d-cis Diltiazem (4 nM) x 60' at 30°C. Final volume was 1 ml. Incubation was stopped by rapid filtration under vacuum through GF/B (0.1% polyethylene) fiber filters which were washed with cold incubation buffer (3 x 3 ml) using the system of filtration Brandel Cell Harvester. Filters were put in 8 ml of Optifluor (Packard) and the radioactivity bound to the membranes trapped by fibers counted by liquid scintillation spectrometer TRI-CARB 1900 CA (Packard). The counting efficiency was about 50%.

### Evaluation of binding data

The percentage inhibition of the specific ³H-ligand binding to its respective receptors was calculated for each concentration (mean of three incubates).

The competition curve, the slope of the curve and IC₅₀^{±} standard deviation values (drug concentration that reduces specific ³H-ligand binding to its respective receptors by 50% of its maximum value) were calculated by using the "Allfit" program (De Lean A. et al., Am. J. Physiol. 235, E97, 1978) running on an IBM 55-SH. The results obtained are shown in Table 1. Compounds resulted inactive at the concentration 10⁻⁵ M have not been tested at higher concentrations.

### Assessment of behaviour and mortality in mice

The assessment of normal behaviour in mice was carried out following S. Irwin's method, Psychopharmacologia 13, 222 (1968). This method allows alterations in some behavioural, neurophysiologic and neurovegetative parameters to be detected, which are directly observable by the researcher. The study was conducted using male CrI: (CD-I)(ICR)BR mice (Charles River - Italy) weighing 22-25 g, following oral administration of the compounds suspended in carboxymethylcellulose (0.5% by weight in H₂O) to groups of 4 animals/dose.

The animals were continuously kept under observation for five hours following treatment and twice a day in the subsequent five days. Mortality was also observed during the overall test period. The initial dose was 1000 mg/kg per os; lower doses were administered in case of death or if the initial dose brought about an excessive response.

The results are shown in Table 2.

### ST 722 studies on isolated organs

The compound was examined in the following preparations of isolated organs:
- Guinea pig ileum
- Rat portal vein

### Isolated guinea pig ileum

### Antagonism to Ca⁺⁺ induced contractions

ST 722 action has been tested in ileal segments K⁺ depolarized and bathed in Ca-free physiological solution at 37°C. A contractile response was evoked by CaCl₂ as described by Spedding M., J. Pharmac. 83, 211 (1984). ST 722 antagonized dose-dependently from 0.1 to 1 µg/ml CaCl₂ contractions.

### Antagonism to angiotensin induced contractions

Ileal segments bathed at 37°C wered made to contract by angiotensin according to the method of Rubin B. et al., J. Pharmacol. Exper. Therap.
204, 271 (1978). ST 722 dose-dependently inhibited angiotensin effect in dose ranging from 0.1 to 1 µg/ml.

### Antagonism to substance P induced contractions

Inhibition to the submaximal contractile response evoked by substance P has been determined in ileum segments bathed at 37°C as described by Holtzer P. et al., Eur. Pharm. 91, 83 (1983). Doses ranging from 0.1 to 1 µg/ml resulted active.

### Antagonsim to methacholine contractions

The compound action has been assayed by using ileum segments made to contract by methacholine chloride according to Magnus R. et al., Physiol. 102, 123 (1904). ST 722 antagonized dose-dependently from 0.1 to 1 µg/ml methacholine contractions.

### Rat portal vein

The effect of the compound on the contractions evoked by high K⁺ concentrations in the rat portal vein has been tested according to Shetty S.S. et al., Europ. J. Pharm. 141, 485 (1989). ST 722 resulted inactive at a concentration of 10 mg/ml.

### Study of the effect on blood pressure and heart rate

The effect of the substance has been tested after oral administration of 100 mg/kg to conscious spontaneously hypertensive rats (SHR). No changes of blood pressure and heart rate were observed 1, 2 and 4 hours after the administration. The experimental procedure was run according to Yeer T.T. et al, Life Sciences 22, 359 (1978).

In conclusion, compounds of the invention resulted active in inhibiting the binding to sites associated to Ca⁺⁺ channels at concentrations of pharmacological interest.

The compounds of the invention, like reference Ca-antagonists, antagonize the contractile activiti of the ileum, but at variance with reference compounds are inactive on contractile activity of portal vein. In addition, they counteracted the contractions evoked by angiotensin, substance P and methacholine.

A pharmacological profile such as shown by these compounds, a Ca-antagonist and anti-cholinergic action in the gastrointestinal tract , but not in other districts (see rat portal vein) in absence of effects on blood pressure "in vivo" is absolutely unexpected.

**Table 1**

| Compound | IC₅₀ | | |
|---|---|---|---|
| | Uptake sites of Ca⁺⁺ channels labelled with | | |
| | ³H-Nitrendipine | ³H-Verapamil | ³H-Diltiazem |
| ST 305 | 3.3 x 10⁻⁵ | 3.5 x 10⁻⁶ | > 10⁻⁵ |
| ST 683 | > 10⁻⁵ | 2.4 x 10⁻⁶ | 1.5 x 10⁻⁵ |
| ST 684 | > 10⁻⁵ | 1.6 x 10⁻⁶ | 7.8 x 10⁻⁶ |
| ST 697 | > 10⁻⁵ | 1.5 x 10⁻⁶ | 5 x 10⁻⁷ |
| ST 712 | 5.3 x 10⁻⁶ | 2.8 x 10⁻⁶ | 1.2 x 10⁻⁶ |
| ST 713 | > 10⁻⁵ | 1.2 x 10⁻⁶ | 1.3 x 10⁻⁶ |
| ST 722 | 1.6 x 10⁻⁶ | 3.8 x 10⁻⁷ | 1.4 x 10⁻⁶ |
| ST 904 | > 10⁻⁵ | 3.2 x 10⁻⁶ | > 10⁻⁵ |

**Table 2**

| Assessment of behaviour and death rate in mice | | | |
|---|---|---|---|
| Compound | Dose | Symptoms | Death rate |
| ST 305 | 1000 | n.e. | 0/4 |
| ST 683 | 300 | n.e. | 0/4 |
| | 600 | convulsions (*) | 2/4 |
| | 1000 | convulsions (*) | 4/4 |
| ST 684 | 1000 | n.e. | 0/4 |
| ST 697 | 100 | n.e. | 0/4 |
| | 300 | convulsions (*) | 1/4 |
| | 600 | convulsions (*) | 2/4 |
| | 1000 | convulsions (*) | 4/4 |
| ST 712 | 1000 | n.e. | 0/4 |
| ST 713 | 100 | n.e. | 0/4 |
| | 300 | convulsions (*) | 2/4 |
| | 600 | convulsions (*) | 3/4 |
| | 1000 | convulsions (*) | 4/4 |
| ST 722 | 1000 | salivation, diarrhea | 0/4 |
| ST 895 | 300 | n.e. | 0/4 |
| | 600 | convulsions (*) | 2/4 |
| | 1000 | convulsions (*) | 4/4 |
| ST 904 | 300 | n.e. | 0/4 |
| | 600 | convulsions (*) | 2/4 |
| | 1000 | convulsions (*) | 4/4 |
| n.e. = no effect | | | |

| | | | |
|---|---|---|---|
| (*) = in connection with death rate only | | | |

## Claims

1. Esters of L-carnitine and acyl L-carnitine of formula (I): 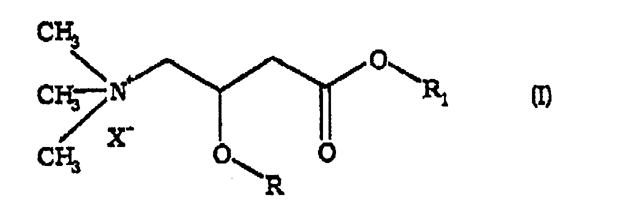 wherein
R is hydrogen or is a straight or branched, saturated or unsaturated acyl group having 2 to 26 carbon atoms;
R₁ is a straight or branched, saturated or unsaturated alkyl group having 4 to 26 carbon atoms; and
X⁻ is the anion of a pharmacologically acceptable acid,
provided that
(1) if R is hydrogen, R₁ is not isobutyl;
(2) if R is C₂-C₁₀ acyl or linoleyl, R₁ is not butyl or isobutyl; and
(3) if R is C₂-C₁₆ acyl, R₁ is not C₁₁-C₁₆ alkyl.

2. Esters according to claim 1, wherein R is
(a) a saturated straight acyl group selected from acetyl, propionyl, butyryl, palmitoyl, undecanoyl and hexacosanoyl; or
(b) a branched acyl group selected from isobutyryl, isovaleryl, isocaproyl and 2-methylhexanoyl; or
(c) 10-undecenoyl.

3. Esters according to claim 1 or 2, wherein R₁ is
(a) a saturated straight alkyl group selected from n-butyl, n-heptyl, n-undecyl and n-hexacosyl; or
(b) a branched alkyl group selected from isobutyl, isooctyl, hexylmethylcarbyl, ethylpentylcarbyl, ethylhexylcarbyl, decylmethylcarbyl, dipentylcarbyl and methylnonylcarbyl; or
(c) an unsaturated alkyl group selected from pentylvinylcabyl and 10-undecenoyl.

4. Esters according to anyone of the preceding claims, wherein X⁻ is selected from chloride; bromide; iodide; aspartate, particularly acid aspartate; citrate, particularly acid citrate; tartrate; phosphate, particularly acid phosphate; fumarate, particularly acid fumarate; glycerophosphate; glucosephosphate; lactate; maleate, particularly acid maleate; orotate; oxalate, particularly acid oxalate; sulphate, particularly acid sulphate; trichloroacetate; trifluoroacetate and methansulfonate.

5. Acteyl L-carnitine heptyl ester chloride.

6. Isobutyryl L-carnitine heptyl ester chloride.

7. Isobutyryl L-carnitine n-butyl ester chloride.

8. Isovaleryl L-carnitine n-butyl ester chloride.

9. Isovaleryl L-carnitine isobutyl ester chloride.

10. Isovaleryl L-carnitine heptyl ester chloride.

11. L-carnitine heptyl ester chloride.

12. Acetyl L-carnitine hexacosyl ester chloride.

13. Isovaleryl L-carnitine undecyl ester chloride.

14. Isobutyryl L-carnitine undecyl ester chloride.

15. An orally or parenterally administrable pharmaceutical composition comprising an ester of anyone of the preceding claims as active ingredient and a pharmacologically acceptable excipient therefor.

16. The composition of claim 15 having muscle relaxant activity selective on the gastrointestinal tract.

17. The composition of claim 15 for treating adaptive colitis syndromes and pathological conditions characterized by increased intestinal contractility and/or motility.

18. Use of an ester of L-carnitine or acyl L-carnitine of formula(I): 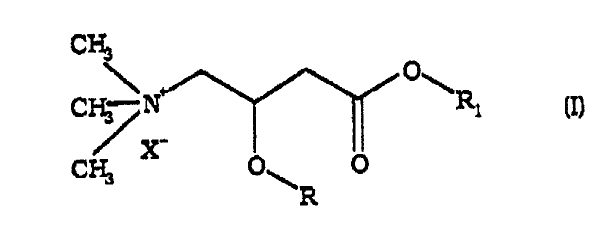 wherein
R is hydrogen or is a straight or branched, saturated or unsaturated acyl group having 2 to 26 carbon atoms;
R₁ is a straight or branched, saturated or unsaturated alkyl group having 4 to 26 carbon atoms; and
X⁻ is the anion of a pharmacologically acceptable acid,
for producing a medicament for treating adaptive colitis syndromes and patholological conditions characterized by increased intestinal contractility an/or motility.

## Patentansprüche

1. Ester von L-Carnitin und Acyl-L-carnitin der Formel (I): 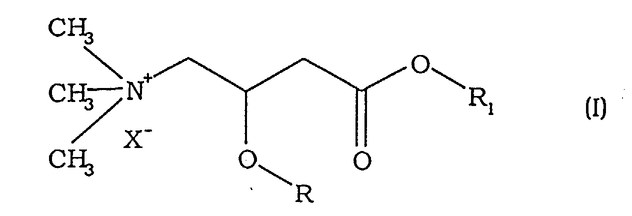 worin:
R Wasserstoff oder eine geradkettige oder verzweigte, gesättigte oder ungesättigte Acyl-Gruppe mit 2 bis 26 Kohlenstoffatomen ist;
R₁ eine geradkettige oder verzweigte, gesättigte oder ungesättigte Alkyl-Gruppe mit 4 bis 26 Kohlenstoffatomen ist; und
X⁻ das Anion einer pharmakologisch akzeptablen Säure ist, vorausgesetzt, daß dann, wenn
(1) R Wasserstoff ist, R₁ nicht Isobutyl ist;
(2) R C₂-C₁₀-Acyl oder Linoleyl ist, R₁ nicht Butyl oder Isobutyl ist; und
(3) R C₂-C₁₆-Acyl ist, R₁ nicht C₁₁-C₁₆-Alkyl ist.

2. Ester nach Anspruch 1, worin R
(a) eine gesättigte, geradkettige Acyl-Gruppe ist, ausgewählt aus Acetyl, Propionyl, Butyryl, Palmitoyl, Undecanoyl und Hexacosanoyl; oder
(b) eine verzweigte Acyl-Gruppe, ausgewählt aus Isobutyryl, Isovaleryl, Isocaproyl und 2-Methylhexanoyl, oder
(c) 10-Undecenoyl.

3. Ester nach Anspruch 1 oder 2, worin R₁
(a) eine gesättigte geradkettige Alkyl-Gruppe ist, ausgewählt aus n-Butyl, n-Heptyl, n-Undecyl und n-Hexacosyl; oder
(b) eine verzweigte Alkyl-Gruppe ist, ausgewählt aus Isobutyl, Isooctyl, Hexylmethylcarbyl, Ethylpentylcarbyl, Ethylhexylcarbyl, Decylmethylcarbyl, Dipentylcarbyl und Methylnonylcarbyl; oder
(c) eine ungesättigte Alkyl-Gruppe ist, ausgewählt aus Pentylvinylcarbyl und 10-Undecenoyl.

4. Ester nach einem der vorhergehenden Ansprüche, worin X⁻ ausgewählt ist aus Chlorid, Bromid, Jodid, Aspartat, insbesondere saures Aspartat, Citrat, insbesondere saures Citrat, Tartrat, Phosphat, insbesondere saures Phosphat, Fumarat, insbesondere saures Fumarat, Glycerophosphat, Glucosephosphat, Lactat, Maleat, insbesondere saures Maleat, Orotat, Oxalat, insbesondere saures Oxalat, Sulfat, insbesondere saures Sulfat, Trichloracetat, Trifluoracetat und Methansulfonat.

5. Acetyl-L-carnitinheptylesterchlorid.

6. Isobutyryl-L-carnitinheptylesterchlorid.

7. Isobutyryl-L-carnitin-n-butylesterchlorid.

8. Isovaleryl-L-carnitin-n-butylesterchlorid.

9. Isovaleryl-L-carnitinisobutylesterchlorid.

10. Isovaleryl-L-carnitinheptylesterchlorid.

11. L-Carnitinheptylesterchlorid.

12. Acetyl-L-carnitinhexacosylesterchlorid.

13. Isovaleryl-L-carnitinundecylesterchlorid.

14. Isobutyryl-L-carnitinundecylesterchlorid.

15. Oral oder parenteral verabreichbare pharmazeutische Zusammensetzung, umfassend einen Ester nach einem der vorhergehenden Ansprüche als aktiven Bestandteil und einen pharmakologisch akzeptablen Exzipienten dafür.

16. Zusammensetzung nach Anspruch 15 mit muskelrelaxierender Aktivität, die für den gastrointestinalen Trakt selektiv ist.

17. Zusammensetzung nach Anspruch 15 zur Behandlung von adaptiven Colitis-Syndromen und pathologischen Zuständen, die durch erhöhte Intestinalkontraktilität und/oder Motilität gekennzeichnet sind.

18. Verwendung eines Esters von L-Carnitin oder Acryl-L-carnitin der Formel (I) : 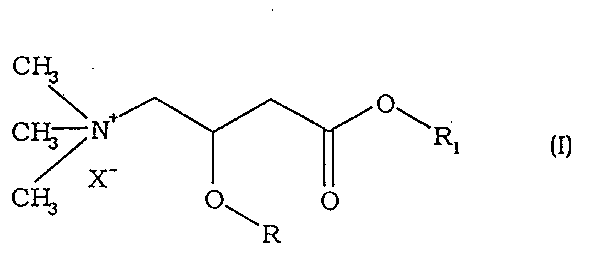 worin:
R Wasserstoff oder eine geradkettige oder verzweigte, gesättigte oder ungesättigte Acyl-Gruppe mit 2 bis 26 Kohlenstoffatomen ist;
R₁ eine geradkettige oder verzweigte, gesättigte oder ungesättigte Alkyl-Gruppe mit 4 bis 26 Kohlenstoffatomen ist; und
X⁻ das Anion einer pharmakologisch akzeptablen Säure ist, zur Herstellung eines Medikamentes zur Behandlung von adaptiven Colitis-Syndromen und pathologischen Zuständen, die durch eine erhöhte intestinale Kontraktilität und/oder Motilität gekennzeichnet sind.

## Revendications

1. Esters de L-carnitine et d'acyl-L-carnitine de formule (I): 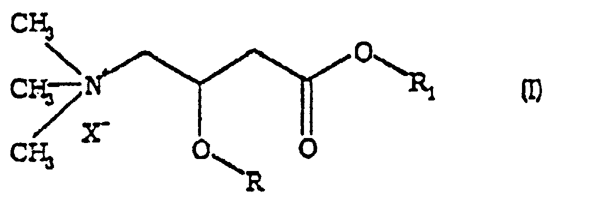 dans laquelle
R est un atome d'hydrogène ou un groupement acyle linéaire ou ramifié, saturé ou insaturé, ayant de 2 à 26 atomes de carbone;
R₁ est un groupement alkyle linéaire ou ramifié, saturé ou insaturé, ayant de 4 à 26 atomes de carbone; et
X⁻ est l'anion d'un acide pharmacologiquement acceptable,
dès lors que
(1) si R est un atome d'hydrogène, R₁ n'est pas un groupement isobutyle;
(2) si R est un groupement acyle en C₂-C₁₀ ou linoléyle, R₁ n'est pas un groupement butyle ou isobutyle; et
(3) si R est un groupement acyle en C₂-C₁₆, R₁ n'est pas un groupement alkyle en C₁₁-C₁₆.

2. Esters selon la revendication 1, caractérisés en ce que R est
(a) un groupement acyle linéaire saturé choisi parmi le groupement acétyle, propionyle, butyryle, palmitoyle, undécanoyle ou hexacosanoyle; ou
(b) un groupement acyle ramifié choisi parmi le groupement isobutyryle, isovaléryle, isocaproyle et 2-méthylhexanoyle; ou
(c) 10-undécénoyle.

3. Esters selon la revendication 1 ou 2, caractérisés en ce que R₁ est
(a) un groupement alkyle linéaire saturé choisi parmi le groupement n-butyle, n-heptyle, n-undécyle et n-hexacosyle; ou
(b) un groupement alkyle ramifié choisi parmi le groupement isobutyle, iso-octyle, hexylméthylcarbyle, éthylpentylcarbyle, éthylhexylcarbyle, décylméthylcarbyle, dipentylcarbyle et méthylnonylcarbyle; ou
(c) un groupement alkyle insaturé choisi parmi le groupement pentylvinylcarbyle et 10-undécénoyle.

4. Esters selon l'une quelconque des revendications précédentes, caractérisés en ce que X⁻ est choisi parmi le chlorure; le bromure; l'iodure; l'aspartate, particulièrement l'aspartate acide; le citrate, particulièrement le citrate acide; le tartrate; le phosphate, particulièrement le phosphate acide; le fumarate, particulièrement le fumarate acide; le glycérophosphate; le glucosephosphate; le lactate; le maléate, particulièrement le maléate acide; l'orotate; l'oxalate, particulièrement l'oxalate acide; le sulfate, particulièrement le sulfate acide; le trichloroacétate; le trifluoroacétate et le méthanesulfonate.

5. Chlorure d'ester heptylique d'acétyl-L-carnitine.

6. Chlorure d'ester heptylique d'isobutyryl-L-carnitine.

7. Chlorure d'ester n-butylique d'isobutyryl-L-carnitine.

8. Chlorure d'ester n-butylique d'isovaléryl-L-carnitine.

9. Chlorure d'ester isobutylique d'isovaléryl-L-carnitine.

10. Chlorure d'ester heptylique d'isovaléryl-L-carnitine.

11. Chlorure d'ester heptylique de L-carnitine.

12. Chlorure d'ester hexacosylique d'acétyl-L-carnitine.

13. Chlorure d'ester undécylique d'isovaléryl-L-carnitine.

14. Chlorure d'ester undécylique d'isobutyryl-L-carnitine.

15. Composition pharmaceutique administrable par voie orale ou parentérale, comprenant un ester de l'une quelconque des revendications précédentes comme principe actif et un excipient pharmacologiquement acceptable pour celle-ci.

16. Composition selon la revendication 15, présentant une activité myorelaxante sélective dans le tractus gastro-intestinal.

17. Composition selon la revendication 15, pour le traitement des syndromes de colite adaptive et des états pathologiques caractérisés par une contractilité et/ou motilité intestinales accrues.

18. Utilisation d'un ester de L-carnitine ou d'acyl-L-carnitine de formule (I): 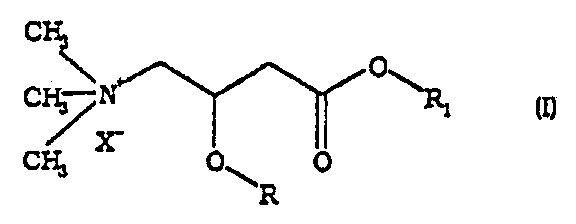 dans laquelle
R est un atome d'hydrogène ou un groupement acyle linéaire ou ramifié, saturé ou insaturé, ayant de 2 à 26 atomes de carbone;
R₁ est un groupement alkyle linéaire ou ramifié, saturé ou insaturé, ayant de 4 à 26 atomes de carbone; et
X⁻ est l'anion d'un acide pharmacologiquement acceptable,
pour la production d'un médicament pour le traitement des syndromes de colite adaptive et des états pathologiques caractérisés par une contractilité et/ou motilité intestinales accrues.
